# EUROPÄISCHE PATENTSCHRIFT

(11) **EP 3 649 974 B1**
(45) Veröffentlichungstag und Bekanntmachung des Hinweises auf die Patenterteilung: **15.09.2021**
(21) Anmeldenummer: 19202505.4
(22) Anmeldetag: 10.10.2019
(51) Int. Cl.: A61B 18/12, A61B 18/14, A61B 18/16, A61B 17/32

(54) **ELEKTRODENANORDNUNG FÜR EIN BIPOLARES RESEKTOSKOP SOWIE RESEKTOSKOP**
ELECTRODE ARRANGEMENT FOR A BIPOLAR RESECTOSCOPE AND RESECTOSCOPE
AGENCEMENT D'ÉLECTRODES POUR UN RÉSECTOSCOPE BIPOLAIRE ET RÉSECTOSCOPE

(30) Priorität: 08.11.2018 DE 102018127919
(43) Veröffentlichungstag der Anmeldung: 13.05.2020
(73) Patentinhaber: Karl Storz SE & Co. KG, 78532 Tuttlingen (DE)
(72) Erfinder: Hahn, Martin, 78532 Tuttlingen (DE); Wittke, Uwe, 78532 Tuttlingen (DE); Hermle, Rainer, 78532 Tuttlingen (DE); Wirth, Franziska, 78532 Tuttlingen (DE)

(56) Entgegenhaltungen:
- US-A- 4 116 198
- US-A- 5 047 027
- US-A1- 2003 233 089
- US-A1- 2015 351 826
- US-B2- 6 471 701

## Beschreibung

Die vorliegende Erfindung betrifft eine Elektrodenanordnung für ein bipolares Resektoskop, die einen lang erstreckten Elektrodenträger, eine an einem distalen Ende des Elektrodenträgers angeordnete Aktivelektrode und eine Neutralelektrode umfasst. Weiter betrifft die Erfindung ein Resektoskop mit einer entsprechenden Elektrodenanordnung.

Elektrodenanordnungen der genannten Art sowie entsprechende Resektoskope sind insbesondere für urologische und gynäkologische Anwendungen bekannt. Hierbei wird die Elektrodenanordnung in ein Arbeitselement des Resektoskops längsverschiebbar eingesetzt und proximalseitig mit einem HF-Generator verbunden. Das Arbeitselement wird zusammen mit der Elektrodenanordnung beispielsweise durch die Harnröhre bis zu einem Operationsgebiet in den Körper eines Patienten eingeführt. Die Aktivelektrode der Elektrodenanordnung wird mit HF-Spannung beaufschlagt und unter endoskopischer Sicht durch einen Gewebebereich geführt. Hierdurch kann Gewebe abgetragen werden, welches mittels einer Spülflüssigkeit abgeführt werden kann. Bei bipolaren Resektoskopen werden sowohl die Aktivelektrode als auch die Neutralelektrode zum Operationsgebiet eingeführt. Dies hat den Vorteil, dass der Stromfluss auf den Bereich zwischen der Aktivelektrode und der Neutralelektrode begrenzt werden kann.

Aus EP 1 567 079 B1 ist ein bipolares medizinisches Instrument zum Schneiden von Gewebe bekannt mit einem sich längs erstreckenden Elektrodenträger, einer an dem Elektrodenträger distalseitig angeordneten, als Drahtschlinge ausgebildeten Aktivelektrode und einer distalseitig der Aktivelektrode benachbart angeordneten Neutralelektrode. Die Neutralelektrode und die Aktivelektrode sind quer zur Längsrichtung des Elektrodenträgers voneinander beabstandet.

Gemäß DE 10 2013 001 156 A1 weist ein bipolares Resektoskop einen Innenschaft mit einem an seinem distalen Ende angeordneten Isoliereinsatz, einen in dem Innenschaft anordenbaren Elektrodentransporteur, eine in dem Elektrodentransporteur längs verschiebliche erste Elektrode und eine zweite Elektrode auf. Der Isoliereinsatz weist an seinem distalen Ende eine umlaufende, elektrisch leitende und quer zur Längsachse des Innenschafts freiliegende Elektrodenfläche auf, die an der Innenseite mit dem distalen Ende der zweiten Elektrode verbunden ist.

In DE 10 2013 109 505 A1 ist ein bipolares medizinisches Instrument zum Schneiden von Gewebe offenbart, wobei an einem distalen Ende eine Aktivelektrode und eine dieser benachbarte Neutralelektrode angeordnet sind und wobei die Neutralelektrode einen gekrümmten Verlauf mit einer ersten und einer zweiten Krümmung hat, wobei die jeweiligen Richtungen der Krümmungen unterschiedlich sind. Dabei kann ein mittlerer Querschnitt und/oder eine Erstreckung der Neutralelektrode größer sein als bei der Aktivelektrode.

Gemäß DE 21 2016 000 007 U1 umfasst eine bipolare Elektrode mit einer linken und rechten Parallelschleife u.a. eine positive Elektrode, zwei Keramikisolierrohre, eine Metallhülse, ein starres Rohr, zwei Isolierrohre und zwei Leitungen. An den vorderen Enden der Metallhülse und des starren Rohrs ist jeweils ein Keramikisolierrohr befestigt, wobei ein Ende der positiven Elektrode im starren Rohr über ein Keramikisolierrohr mit einer Leitung verbunden und das andere Ende in dem der Metallhülse zugeordneten Keramikisolierrohr gestützt ist. Die andere Leitung ist mit der zugeordneten Metallhülse angeschweißt. Das starre Rohr dient dabei nicht als Schleifenelektrode.

US 6471701 B2 offenbart eine Elektrodenanordnung für ein Resektoskop. Die Anordnung umfasst: einen langgestreckten Elektrodenträger, einen Elektrodenkörper/Neutralelektrode und eine Aktivelektrode. Der Elektrodenkörper und der Elektrodenträger sind über die Stromleitung der Aktivelektrode und der Neutralelektrode verbunden.

US 5047027 A offenbart eine Elektrodenanordnung für ein Resektoskop. Das System umfasst einen Elektrodenträger, einen Elektrodenkörper/Neutralelektrode, und eine Aktivelektrode. US5047027 offenbart, dass der Elektrodenkörper und der Elektrodenträger im Wesentlichen den gleichen Querschnitt haben.

Bei den bekannten Elektrodenanordnungen für bipolare Resektoskope verursacht die Neutralelektrode einen zusätzlichen Platzbedarf und/oder eine Verringerung der mechanischen Stabilität der Elektrodenanordnung. Ferner stellt die Neutralelektrode in der Regel ein zusätzliches Bauteil dar, wodurch ein erhöhter Fertigungs- und Montageaufwand entsteht.

Es ist Aufgabe der vorliegenden Erfindung, eine verbesserte Elektrodenanordnung für ein bipolares Resektoskop anzugeben, wobei die oben genannten Nachteile möglichst vermieden werden, insbesondere wobei der durch die Neutralelektrode verursachte Raumbedarf und/oder der Fertigungs- bzw. Montageaufwand verringert ist. Weiter ist es Aufgabe der Erfindung, ein entsprechendes Resektoskop anzugeben.

Diese Aufgabe wird durch eine Elektrodenanordnung gemäß Anspruch 1 sowie durch ein Resektoskop gemäß Anspruch 13 gelöst.

Vorteilhafte Weiterbildungen der Erfindung ergeben sich aus den Unteransprüchen.

Eine erfindungsgemäße Elektrodenanordnung ist zur Verwendung in einem bipolaren Resektoskop ausgebildet, insbesondere für urologische oder gynäkologische Anwendungen. Die Elektrodenanordnung umfasst einen lang erstreckten Elektrodenträger, eine Aktivelektrode und eine Neutralelektrode, wobei die Aktivelektrode an einem distalen Ende des Elektrodenträgers angeordnet ist. Die Neutralelektrode ist ebenfalls im distalen Endbereich des Elektrodenträgers angeordnet. Der Elektrodenträger ist dafür ausgebildet, in eincm Schaft des Rcscktoskops aufgenommen zu werden und mittels eines Verschiebemechanismus in Längsrichtung des Schafts verschoben zu werden. Der Elektrodenträger umfasst Zuleitungen für die Aktivelektrode und für die Neutralelektrode; die Zuleitungen sind typischerweise bis zu einem proximalen Ende des Elektrodenträgers geführt und enden dort in Steckverbindern, die zur elektrischen Verbindung mit einem HF-Anschluss des Resektoskops dienen. Die Aktivelektrode kann beispielsweise als Drahtschlinge oder auch in anderer Form ausgebildet sein und ragt vorzugsweise in distaler und/oder seitlicher Richtung über den Elektrodenträger hinaus. Eine solche Elektrodenanordnung wird oft auch kurz als "Elektrode" bezeichnet.

Erfindungsgemäß ist ein distaler Endabschnitt des Elektrodenträgers als ein Elektrodenkörper ausgebildet, welcher insbesondere einstückig ausgebildet sein kann. Durch den Elektrodenkörper ist eine Zuleitung der Aktivelektrode geführt, insbesondere verläuft die Zuleitung der Aktivelektrode von einer proximalen Seite des Elektrodenkörpers durch diesen hindurch bis zu einer distalen Seite des Elektrodenkörpers, die beispielsweise durch eine distale Stirnfläche des Elektrodenkörpers gebildet werden kann. Der Elektrodenkörper kann als Hohlkörper oder auch, bis auf ggf. vorhandene Bohrungen etwa für Zuleitungen, massiv ausgebildet sein.

Erfindungsgemäß wird die Neutralelektrode durch den Elektrodenkörper oder durch einen Teil oder einen Teilbereich des Elektrodenkörpers gebildet, insbesondere durch einen solchen Teil oder Teilbereich des Elektrodenkörpers, der eine äußere Oberfläche des Elektrodenkörpers oder einen Teilbereich einer äußeren Oberfläche bildet. Zumindest derjenige Teil oder Teilbereich des Elektrodenkörpers, der als Neutralelektrode dient, ist hierfür elektrisch leitfähig ausgebildet und mit einer Zuleitung der Neutralelektrode elektrisch verbunden. Die Neutralelektrode kann vollständig oder überwiegend proximalseitig der Aktivelektrode angeordnet sein. Ferner ist die wirksame Fläche der Neutralelektrode vorzugsweise größer als eine Oberfläche der Aktivelektrode.

Die Elektrodenanordnung kann weitere Elemente umfassen, etwa Führungsklammern zur Führung des Elektrodenträgers innerhalb eines Schafts des Resektoskops oder am proximalen Ende des Elektrodenträgers angeordnete Steckverbinder zum Verbinden der Zuleitungen mit entsprechenden Kontakten und/oder zum Arretieren der Elektrodenanordnung in dem Verschiebemechanismus des Resektoskops.

Bei der Anwendung der Elektrodenanordnung werden die Elektroden mit HF-Spannung beaufschlagt. Dabei findet ein Stromfluss zwischen der Aktivelektrode und der Neutralelektrode statt, so dass mit der Aktivelektrode eine elektrochirurgische Abtragung oder ein Durchtrennen von Gewebe sowie ggf. eine Koagulation erfolgen kann. Die Aktivelektrode kann hierfür durch Längsverschiebung des Elektrodenträgers gemeinsam mit der Neutralelektrode verschoben werden.

Dadurch, dass der distale Endabschnitt des Elektrodenträgers durch einen Elektrodenkörper gebildet wird, welcher selbst oder zumindest ein Teil dessen die Neutralelektrode darstellt, kann eine Neutralelektrode geschaffen werden, die keinen oder nur einen minimalen zusätzlichen Raumbedarf hat. Insbesondere kann dadurch, dass die Zuleitung der Aktivelektrode durch den Elektrodenkörper geführt ist, eine besonders raumsparende Anordnung geschaffen werden, wobei die Neutralelektrode zugleich eine besonders große wirksame Fläche aufweist. Als weiterer Vorteil kann der Abstand Aktiv- zu Passiv- bzw. Neutralelektrode vergrößert werden. Hierdurch kann einerseits eine günstige Raumausnutzung innerhalb des Schafts eines Resektoskops erreichbar sein, wobei ein großes Lumen für weitere Kanäle sowie für eine Endoskopoptik zur Verfügung steht, und andererseits ein sicheres Schneiden ermöglicht werden, wobei das Gewebe nur bei Berührung mit der Aktivelektrode geschnitten wird. Weiterhin kann sich die Passiv- bzw. Neutralelektrode nicht mehr im Sichtfeld befinden und das Sichtfeld damit verbessert werden. Zudem kann durch die erfindungsgemäße Ausbildung der Neutralelektrode diese als ein kompaktes Bauteil ausgebildet sein, das eine höhere Stabilität gewährleistet. Schließlich kann auf diese Weise die Montage der Elektrodenanordnung vereinfacht werden, da die Neutralelektrode kein separates Bauteil darstellt, sondern gemeinsam mit dem Elektrodenkörper montiert werden kann.

Erfindungsgemäß umfasst der Elektrodenträger einen lang erstreckten Elektrodenschaft, dessen Länge näherungsweise einer Länge eines Resektoskopschafts entsprechen kann, und der distale Endabschnitt des Elektrodenträgers bzw. der Elektrodenkörper ist in einer distalen Fortsetzung des Elektrodenschafts angeordnet, wobei ein Querschnitt des Elektrodenkörpers bezüglich Form und Größe zumindest näherungsweise einem Querschnitt des Elektrodenschafts entspricht. Hierdurch kann eine optimale Ausnutzung der Raumverhältnisse innerhalb des Schafts des Resektoskops erreichbar sein.

In bevorzugter Weise sind der Elektrodenschaft und der Elektrodenkörper voneinander abgesetzt und durch einen Zwischenraum voneinander getrennt. Vorzugsweise sind der Elektrodenschaft und der Elektrodenkörper durch die Zuleitung der Aktivelektrode und die Zuleitung der Neutralelektrode miteinander verbunden, insbesondere nur durch die genannten Zuleitungen, wobei die Zuleitungen zumindest in dem Zwischenraum zwischen dem Elektrodenkörper und dem Elektrodenschaft jeweils von einer Isolierhülle umgeben sein können. Hierdurch kann zugleich eine Oberfläche des Elektrodenschafts gegen die Zuleitungen sowie gegen die Neutralelektrode isoliert sein. Die Verbindungsabschnitte der Zuleitungen, die in dem Zwischenraum liegen, können dabei gleich lang oder unterschiedlich lang ausgebildet sein. Hierdurch kann ein besonders einfacher Aufbau erreicht werden.

In besonders bevorzugter Weise kann zudem die Zuleitung der Neutralelektrode proximalseitig in den Elektrodenkörper eingesetzt sein. Hierdurch kann eine einfache und kompakte Bauform erzielt werden, und es kann ferner ermöglicht werden, auf besonders einfache und sichere Weise eine elektrische Verbindung der Zuleitung mit der Neutralelektrode zu erreichen.

Gemäß einer besonders bevorzugten Ausführungsform der Erfindung ist der Elektrodenkörper als ein Isolierkörper mit einer elektrisch leitfähigen Schicht ausgebildet, wobei die Zuleitung der Aktivelektrode durch den Isolierkörper geführt ist. Gemäß diesem Aspekt der Erfindung ist der Elektrodenkörper somit ganz oder weitgehend aus einem elektrisch nicht leitfähigen Material aufgebaut, und die wirksame Fläche der Neutralelektrode wird durch die elektrisch leitfähige Schicht gebildet, die zumindest auf einen Teilbereich einer Oberfläche des Isolierkörpers aufgebracht ist. Der Isolierkörper dient somit als Grundkörper, auf den zumindest teilweise die elektrisch leitfähige Schicht aufgetragen ist. Dadurch, dass der distale Endabschnitt des Elektrodenträgers durch einen Isolierkörper gebildet wird, durch den die Zuleitung der Aktivelektrode verläuft und auf dessen Oberfläche die Neutralelektrode durch eine elektrisch leitfähige Schicht gebildet wird, kann auf einfache Weise bewirkt werden, dass die Aktivelektrode von der Neutralelektrode elektrisch isoliert ist. Weiter kann der Isolierkörper mit der leitfähigen Schicht als kompaktes Bauteil ausgebildet sein, das einfach herzustellen und leicht zu montieren ist und das eine besonders hohe Stabilität gewährleistet.

Vorzugsweise besteht der Isolierkörper ganz oder weitgehend aus einem keramischen Material, ggf. auch einem anderen HF- und temperaturbeständigen Material. Dieses hat den besonderen Vorteil, dass es nicht nur eine sichere Isolierung der durch den Isolierkörper geführten Zuleitung der Aktivelektrode gewährleisten, sondern auch eine hohe mechanische, thermische und chemische Stabilität aufweisen kann. Ein derartiger keramischer Isolierkörper kann in einfacher und kostengünstiger Weise in einer geeigneten Form, beispielsweise als Hohlkörper oder auch als massiver Körper mit Bohrungen, hergestellt werden.

In bevorzugter Weise ist die elektrisch leitfähige Schicht eine elektrisch leitfähige Beschichtung, insbesondere eine metallische Beschichtung. Diese kann mit einem üblichen Beschichtungsverfahren etwa als dünne Schicht auf die Oberfläche des Isolierkörpers aufgebracht werden. Hierdurch wird sowohl eine besonders einfache Herstellung als auch eine besonders stabile Ausführung der Elektrodenanordnung ermöglicht. Alternativ kann die elektrisch leitfähige Schicht eine dicke Schicht sein, die beispielsweise durch ein auf die Oberfläche des Isolierkörpers aufgebrachtes und der Form der Oberfläche angepasstes Bauteil, etwa eine Ummantelung, gebildet werden kann.

Vorzugsweise ist derjenige Teilbereich der Oberfläche des Isolierkörpers, auf den die elektrisch leitfähige Schicht bzw. die Beschichtung aufgebracht ist, die die wirksame Fläche der Neutralelektrode bildet, ein solcher Teilbereich, der eine seitliche, insbesondere eine umlaufende Oberfläche des Isolierkörpers oder einen Teilbereich derselben darstellt. Die wirksame Fläche der Neutralelektrode ist somit vorzugsweise eine seitliche Oberfläche des Elektrodenkörpers. Als seitliche Oberfläche wird hier eine Oberfläche bezeichnet, die in Bezug auf eine Längsachse des Elektrodenträgers bzw. des Elektrodenkörpers seitlich angeordnet ist, insbesondere eine solche Oberfläche, deren Flächennormalen quer zur Längsachse gerichtet sind. Hierdurch können eine einfache Bauform und eine kostengünstige Herstellung wie auch eine sichere Isolierung und eine für die Anwendung günstige Stromverteilung erreichbar sein.

In vorteilhafter Weise kann der Isolierkörper zumindest näherungsweise als ein Zylinder oder ein abgeflachter Zylinder ausgebildet sein, wobei zumindest ein Teilbereich der Mantelfläche des Zylinders als Neutralelektrode und hierfür mit der elektrisch leitfähigen Schicht bzw. der Beschichtung belegt ist. Der abgeflachte Zylinder sowie die auf die Oberfläche aufgebrachte Schicht können zumindest abschnittsweise spiegelsymmetrisch zu einer Längsmittelebene oder achsensymmetrisch zu der Längsmittelachse des Elektrodenträgers ausgebildet sein. Hierdurch wird ein besonders einfacher und für die Anwendung günstiger Aufbau erreicht.

Gemäß einer vorteilhaften Ausführungsform der Erfindung ist eine Zuleitung der Neutralelektrode proximalseitig in den Isolierkörper eingesetzt, und der Isolierkörper weist eine Querbohrung auf, die bis zu der in den Isolierkörper eingesetzten Zuleitung der Neutralelektrode reicht und in die sich die Schicht bzw. die elektronisch leitende, insbesondere metallische Beschichtung bis zur Oberfläche der Zuleitung erstreckt. Hierdurch kann auf besonders einfache Weise der elektrische Kontakt zwischen der Schicht bzw. der elektronisch leitenden, insbesondere metallischen Beschichtung, die die Neutralelektrode bildet, und der Zuleitung hergestellt werden.

In besonders bevorzugter Weise ist die Aktivelektrode als Schneidschlinge mit einem ersten und einem zweiten Fußabschnitt sowie einem dazwischenliegenden Schneidabschnitt ausgebildet, wobei der erste und der zweite Fußabschnitt jeweils an einer distalen Stirnfläche des Isolierkörpers ansetzen, welche Stirnfläche insbesondere nicht die elektrisch leitfähige Schicht aufweist. Der erste Fußabschnitt ist dabei mit der Zuleitung der Aktivelektrode verbunden bzw. mit dieser durchgehend ausgebildet, während der zweite Fußabschnitt ohne elektrische Verbindung mit einer Zuleitung in den Isolierkörper eingesetzt ist. Die Aktivelektrode ist somit nur über ihren ersten Fußabschnitt mit der Zuleitung elektrisch verbunden, während der zweite Fußabschnitt durch den Isolierkörper gestützt, aber durch diesen von der Neutralelektrode und deren Zuleitung isoliert ist. Hierdurch kann eine besonders einfache, stabile und leicht handhabbare Aktivelektrode geschaffen werden.

Alternativ kann die Aktivelektrode beispielsweise stabförmig mit nur einem einzigen Fußabschnitt ausgebildet sein, der an der distalen Stirnfläche des Isolierkörpers ansetzt und mit der Zuleitung der Aktivelektrode verbunden ist bzw. in diese übergeht, wobei der Stab vorzugsweise quer zur Längsachse des Elektrodenkörpers bzw. des Elektrodenschafts gerichtet ist. Zusätzlich kann die Aktivelektrode an dem vom Fußabschnitt abgewandten Ende des Stabs eine metallische Kugel oder ein anderes Element mit einer geeigneten Elektrodenfläche aufweisen. Eine derartig ausgebildete Elektrodenanordnung ist ebenfalls einfach und stabil sowie beispielsweise zum Schneiden und/oder Koagulieren von Gewebe geeignet.

Gemäß einer weiteren bevorzugten Ausführungsform der Erfindung ist der distale Endabschnitt des Elektrodenträgers als metallischer Elektrodenkörper ausgebildet, der die Neutralelektrode bildet und durch den die Zuleitung der Aktivelektrode isoliert geführt ist. Insbesondere ist die Zuleitung der Aktivelektrode von einer proximalen Seite zu einer distalen Seite des metallischen Elektrodenkörpers isoliert durch diesen hindurchgeführt und hierfür beispielsweise von einer Isolierhülle umgeben. Die Isolierhülle kann sich in proximaler und/oder in distaler Richtung über den metallischen Elektrodenkörper hinaus erstrecken. Der metallische Elektrodenkörper kann beispielsweise näherungsweise in Form eines Zylinders oder eines abgeflachten Zylinder ausgebildet sein. Auch ein derartiger metallischer Elektrodenkörper kann in einfacher und kostengünstiger Weise in einer geeigneten Form, beispielsweise als Hohlkörper oder auch als massiver Körper mit Bohrungen, hergestellt werden. Hierdurch kann ebenfalls eine besonders einfache, platzsparende und stabile Elektrodenanordnung geschaffen werden.

In vorteilhafter Weise kann die Aktivelektrode als Schneidschlinge mit einem ersten und einem zweiten Fußabschnitt und einem dazwischenliegenden Schneidabschnitt ausgebildet sein, wobei der erste und der zweite Fußabschnitt an einem distalen Ende der Zuleitung der Aktivelektrode ansetzen. Insbesondere kann eine Isolierhülle, die die Zuleitung der Aktivelektrode innerhalb des metallischen Elektrodenkörpers umgibt und die Aktivelektrode gegen die Neutralelektrode elektrisch isoliert, distalseitig über eine distale Stirnfläche des metallischen Elektrodenkörpers hinaus ragen, wobei zumindest der erste Fußabschnitt im distalen Endbereich der Isolierhülle elektrisch mit der Zuleitung verbunden ist bzw. in diese übergeht und hierdurch zugleich mechanisch gestützt wird. Hierdurch kann eine einfache, wirksame und leicht handhabbare Elektrodenanordnung geschaffen werden.

Alternativ kann die Aktivelektrode stabförmig und mit nur einem einzigen Fußabschnitt ausgebildet sein, der im distalen Endbereich der Isolierhülle mit der Zuleitung der Aktivelektrode verbunden ist. Im Übrigen kann die Aktivelektrode gemäß dieser Ausführungsform wie die oben beschriebene stabförmige Elektrode ausgebildet sein und beispielsweise zusätzlich an dem vom Fußabschnitt abgewandten Ende des Stabs eine metallische Kugel oder ein anderes Element mit einer geeigneten Elektrodenfläche tragen.

Ein erfindungsgemäßes Resektoskop umfasst einen lang erstreckten Schaft, eine in diesen längsverschiebbar eingesetzte, erfindungsgemäß ausgebildete Elektrodenanordnung und einen Verschiebemechanismus zum Verschieben der Elektrodenanordnung in der Längsrichtung des Schafts. Der Schaft kann ferner zur Aufnehmen einer Endoskopoptik ausgebildet sein. Der Schaft mit dem Verschiebemechanismus wird üblicherweise auch als "Arbeitselement" bezeichnet, und der Zusammenbau aus dem Arbeitselement mit der eingesetzten Elektrodenanordnung üblicherweise und in der vorliegenden Anmeldung als "Resektoskop"; gelegentlich wird die Bezeichnung "Resektoskop" auch für das Arbeitselement alleine oder für den Zusammenbau aus Arbeitselement, Elektrode und Endoskopoptik verwendet.

Der Schaft des Resektoskops ist insbesondere zur Einführung in eine Körperöffnung eines menschlichen oder tierischen Körpers, beispielsweise zur Einführung in die Harnröhre eines Menschen, bemessen und kann einen als langerstrecktes Hohlrohr ausgebildeten Außenschaft sowie einen in diesen eingesetzten und mit dem Außenschaft über einen Kupplungsmechanismus verbundenen Innenschaft umfassen. Typischerweise ist am proximalen Ende des Innenschafts eine Handhabe angeordnet, die den Verschiebemechanismus zum axialen Verschieben der in den Innenschaft eingesetzten Elektrodenanordnung aufweist. Am proximalen Endbereich des Schafts bzw. an der Handhabe können weiterhin ein Spülanschluss, ein Sauganschluss sowie ein oder mehrere elektrische Anschlüsse und eine Optikkupplung zum Fixieren der in den Schaft eingeführten Endoskopoptik vorgesehen sein.

Der Elektrodenträger der Elektrodenanordnung kann an seinem proximalen Ende Steckverbinder aufweisen, über die Aktiv- und die Neutralelektrode mit einem HF-Anschluss des Resektoskops kontaktierbar sind, um die Zuleitungen der Aktivelektrode und der Neutralelektrode mit einem externen HF-Generator, der die benötigte HF-Spannung erzeugt, zu verbinden. Ferner kann der Elektrodenträger in dem Verschiebemechanismus arretierbar sein, um eine kontrollierbare Längsverschiebung zu ermöglichen.

Weitere Aspekte der Erfindung ergeben sich aus der nachfolgenden Beschreibung bevorzugter Ausführungsbeispiele und der beigefügten Zeichnung. Es zeigen:
Fig. 1 eine Elektrodenanordnung gemäß einem ersten Ausführungsbeispiel der Erfindung in einer Gesamtansicht;
Fig. 2 den distalen Endbereich der Elektrodenanordnung aus Fig. 1 in einer Schrägansicht;
Fig. 3 den distalen Endbereich der Elektrodenanordnung aus Fig. 1 in einer teilweise transparenten Darstellung;
Fig. 4 den distalen Endbereich einer Elektrodenanordnung gemäß einem zweiten Ausführungsbeispiel der Erfindung;
Fig. 5 den distalen Endbereich einer Elektrodenanordnung gemäß einem dritten Ausführungsbeispiel der Erfindung;
Fig. 6 den distalen Endbereich einer Elektrodenanordnung gemäß einem vierten Ausführungsbeispiel der Erfindung;
Fig. 7 ein Resektoskop mit einer Elektrodenanordnung gemäß Fig. 1.

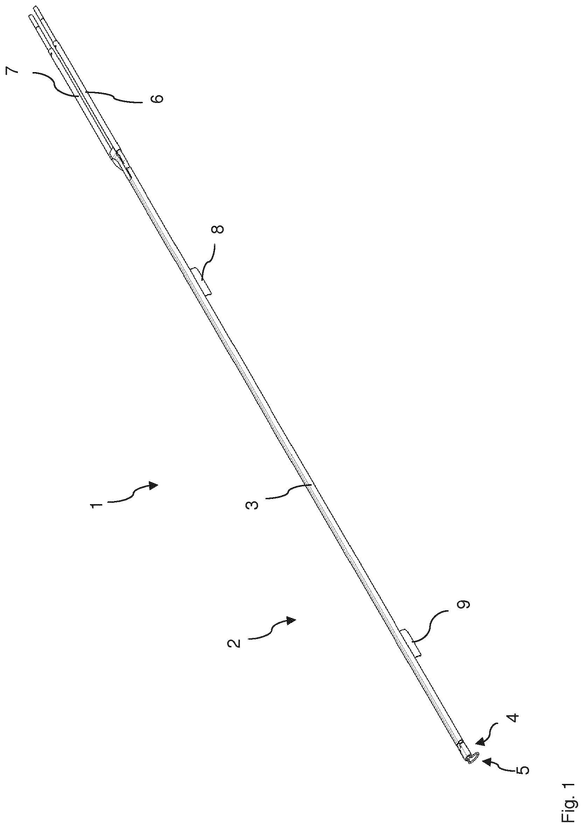

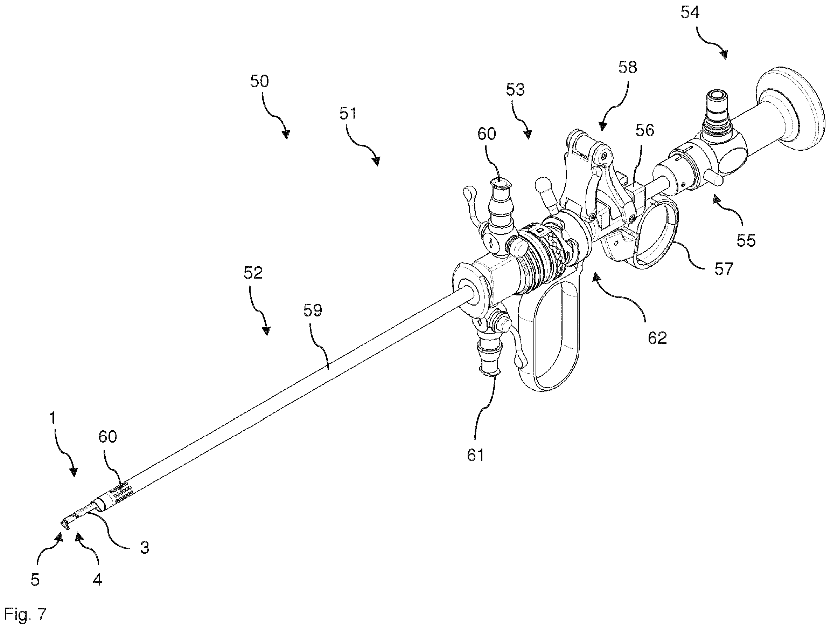

In Fig. 1 ist exemplarisch eine Elektrodenanordnung gemäß einem ersten Ausführungsbeispiel der Erfindung in einer Gesamtansicht dargestellt. Die Elektrodenanordnung 1 umfasst einen Elektrodenträger 2, der einen langerstreckten Elektrodenschaft 3 und einen an dessen distalem Ende angeordneten Elektrodenkörper 4 aufweist. Distalseitig ist an dem Elektrodenkörper 4 eine als Schneidschlinge 5 ausgebildete Aktivelektrode angesetzt. Proximalseitig sind am Elektrodenschaft 3 zwei parallele Steckverbinder 6, 7 vorgesehen, die zum Herstellen einer elektrischen Verbindung mit entsprechenden elektrischen Kontakten des Arbeitselements eines Resektoskops dienen und auch für eine mechanische Arretierung in dem Verschiebemechanismus des Resektoskops ausgestaltet sein können. An dem Elektrodenschaft 3 sind Führungsklammern 8, 9 zur Führung innerhalb eines Schafts des Arbeitselements des Resektoskops (s. Fig. 7) angebracht.

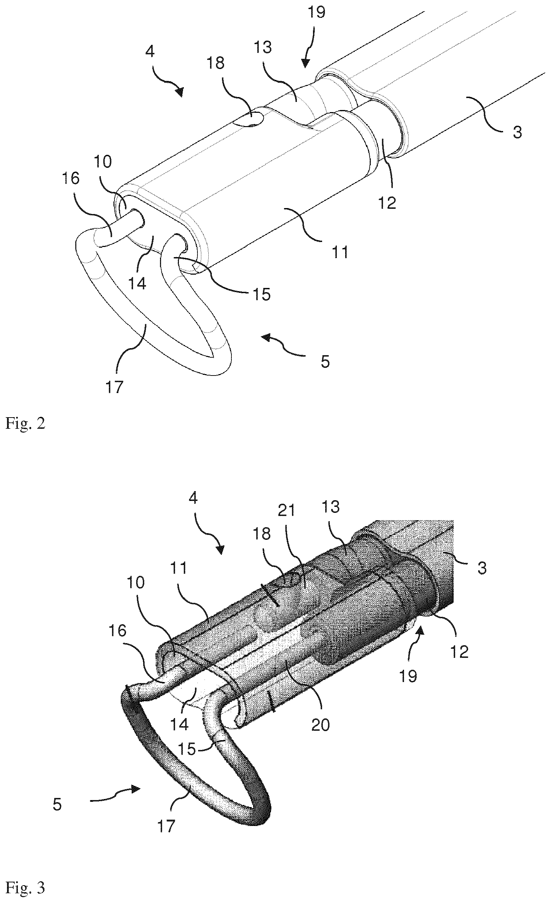

In Fig. 2 ist der distale Endbereich der Elektrodenanordnung 2 in einer vergrößerten Ansicht, schräg aus distaler Richtung gesehen, gezeigt. Am distalen Ende des Elektrodenschafts 3 ist in gerader Fortsetzung des Elektrodenschafts 3 der Elektrodenkörper 4 angeordnet. Dieser weist näherungsweise einen gleichen Querschnitt wie der Elektrodenschaft 3 auf. Im dargestellten Ausführungsbeispiel haben der Elektrodenschaft 3 und der distale Endabschnitt jeweils näherungsweise die Form eines abgeflachten Zylinders.

Der Elektrodenkörper 4 wird durch einen keramischen Isolierkörper 10 gebildet, dessen Oberfläche teilweise von einer elektronisch leitenden, insbesondere metallischen, Beschichtung 11 bedeckt ist. Die Beschichtung 11 bedeckt die Oberseite der Mantelfläche des näherungsweise abgeflacht zylindrisch geformten Isolierkörpers 10 und greift etwas über dessen Mitte nach unten hinaus. Die Beschichtung 11 kann mit üblichen Beschichtungsverfahren auf die entsprechenden Oberflächenbereiche des Isolierkörpers aufgebracht worden sein. Die Stirnseiten des Isolierkörpers 10 sind nicht mit der Beschichtung 11 belegt. Proximalseitig ist der Elektrodenkörper 4 mit dem Elektrodenschaft über zwei Zuleitungen verbunden, die jeweils von einer Isolierhülle 12, 13 umgeben sind. An der distalen Stirnfläche 14 ist die als Schneidschlinge 5 ausgebildete Aktivelektrode angesetzt. Bei dem in Fig. 2 dargestellten Ausführungsbeispiel weist die Schneidschlinge einen ersten Fußabschnitt 15 und einen zweiten Fußabschnitt 16 auf, mit denen sie an der distalen Stirnfläche 14 des Isolierkörpers 10 angesetzt ist, sowie einen dazwischenliegenden Schneidabschnitt 17, der über eine distalseitige Verlängerung des Querschnitts des Elektrodenkörpers 4 bzw. des Elektrodenschafts 3 nach unten hinaus ragt und der zum Schneiden von Gewebe mit diesem in Berührung gebracht wird. Der Isolierkörper 10 weist eine Querbohrung 18 auf, in die sich die Beschichtung 11 hinein erstreckt (siehe unten).

Der Elektrodenkörper 4 ist in Fig. 3 in teiltransparenter Darstellung gezeigt. Wie in Fig. 3 zu erkennen ist, geht der erste Fußabschnitt 15 der Schneidschlinge 5 innerhalb des Isolierkörpers 10 in eine Zuleitung 20 über. Die Zuleitung 20 vermittelt die elektrische Verbindung der Schneidschlinge 5 mit einem HF-Anschluss des Resektoskops und ist durch den Elektrodenschaft 3 bis in den Steckverbinder 6 geführt (s. Fig. 1). Die Zuleitung 20 ist innerhalb des Elektrodenschafts sowie in einem Zwischenraum 19 zwischen dem Elektrodenschaft 3 und dem Elektrodenkörper 4 von der Isolierhülle 12 umgeben, die sich auch ein Stück weit in den Elektrodenkörper 4 hinein erstreckt. Der zweite Fußabschnitt 16 der Schneidschlinge 5 ist in den Isolierkörper 10 eingesetzt, jedoch nicht elektrisch kontaktiert. Die Schneidschlinge 5 kann mit der Zuleitung 20 als durchgehender Draht ausgebildet sein.

Durch den Elektrodenschaft 3 verläuft eine weitere Zuleitung 21, die die elektrische Verbindung zwischen der durch die Beschichtung 11 gebildeten Neutralelektrode und einem entsprechenden HF-Anschluss des Resektoskops über den Verbindungsstecker 7 vermittelt (s. Fig. 1). Wie in Fig. 3 zu erkennen ist, ist die Zuleitung 21 der Neutralelektrode in dem Zwischenraum 19 zwischen dem Elektrodenschaft und dem distalen Endabschnitt von der Isolierhülle 13 umgeben und erstreckt sich ein Stück weit in den Isolierkörper 10 hinein. Die Querbohrung 18 reicht bis zur Oberfläche der Zuleitung 21. Die Beschichtung 11 erstreckt sich in die Querbohrung hinein bis zur Zuleitung 21 und stellt damit den elektrischen Kontakt mit der Zuleitung 21 her. Wie in Fig. 2 angedeutet ist, ist die proximale Stirnseite des Elektrodenkörpers 4 abgestuft ausgebildet, wobei die Zuleitung 20 der Aktivelektrode den Zwischenraum 19 mit einem kürzeren Abschnitt überbrückt, während die Zuleitung 21 der Neutralelektrode mit einem längeren Abschnitt den Zwischenraum 19 überbrückt, um einen Querversatz zwischen den Zuleitungen 20, 21 auszugleichen.

Durch die beschriebene Anordnung wird einerseits eine Versorgung der Schneidschlinge 5 mit der zum Schneiden erforderlichen HF-Energie und andererseits eine Verbindung der Beschichtung 11, die die wirksame Fläche der Neutralelektrode darstellt, mit der Zuleitung 21 hergestellt. Zugleich wird durch das keramische Material des Isolierkörpers 10, in den die Zuleitungen 20, 21 und die Fußabschnitte 15, 16 der Schneidschlinge 5 eingebettet sind, eine elektrische Trennung der Aktiv- und der Neutralelektrode gewährleistet. Schließlich dient der Isolierkörper 10 zum Stützen der Schneidschlinge 5 und als Grundkörper zum Auftragen der Beschichtung 11.

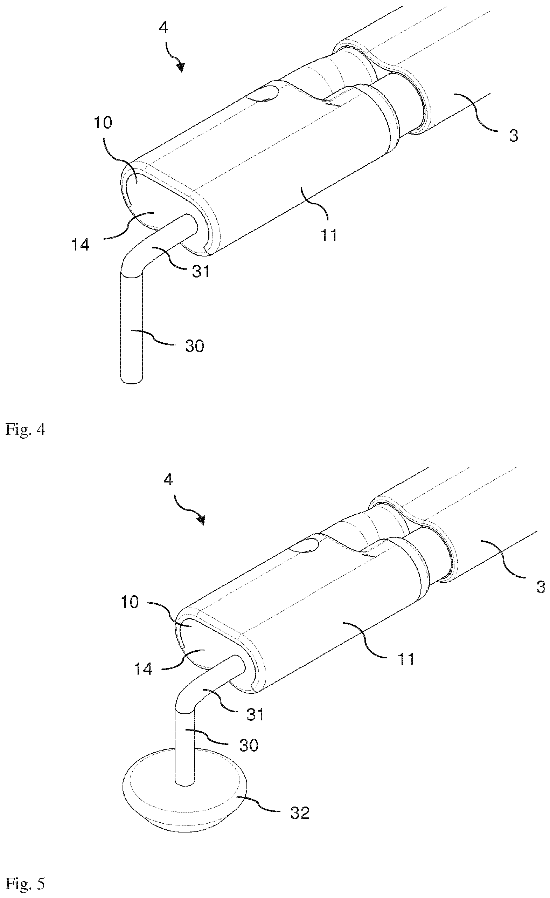

In der Figur 4, die eine der Figur 2 entsprechende Ansicht zeigt, ist der distale Endbereich der Elektrodenanordnung gemäß einem weiteren Ausführungsbeispiel der Erfindung dargestellt. Bei diesem Ausführungsbeispiel ist die Schneidelektrode in Form eines sich quer zur Längsrichtung des Elektrodenschafts 3 und des distalen Endabschnitts 4 erstreckenden metallischen Stabs 30 ausgebildet. Der Stab 30 ist über den Fußabschnitt 31 an die distale Stirnfläche 14 des Isolierkörpers 10 angesetzt und mit der Zuleitung 20 verbunden, wobei der Stab 30, der Fußabschnitt 31 und die Zuleitung 20 durch einen durchgehenden Draht gebildet werden können. Im Übrigen ist die Elektrodenanordnung gemäß diesem Ausführungsbeispiel wie die zuvor anhand der Figuren 1 bis 3 beschriebene ausgebildet.

Eine dritte Ausführungsform der erfindungsgemäßen Elektrodenanordnung ist in Fig. 5 gezeigt. Hierbei ist die Schneidelektrode wie die in Fig. 4 gezeigte mit einem Stab 30 und einem einzigen Fußabschnitt 31 ausgebildet, wobei der Stab 30 zusätzlich an seinem vom Fußabschnitt 31 abgewandten Ende eine metallische Kugel 32 trägt. Anstelle der Kugel 32 kann auch beispielsweise eine Halbkugel oder eine Scheibe vorgesehen sein. Im Übrigen ist diese Ausführungsform wie zuvor beschrieben ausgebildet.

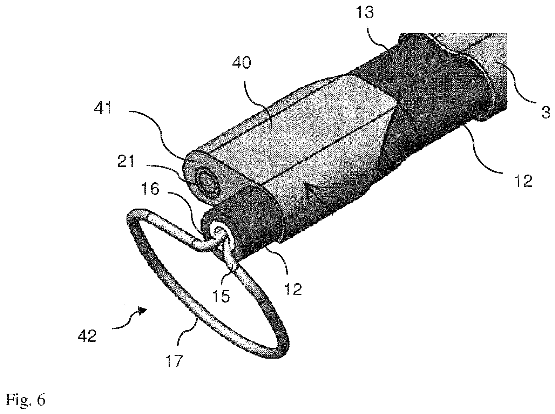

Bei der in Fig. 6 dargestellten weiteren Ausführungsform der Erfindung wird der distale Endabschnitt des Elektrodenträgers 2 nicht, wie bei den zuvor beschriebenen Ausführungsbeispielen, durch einen mit einer elektronisch leitenden, insbesondere metallischen Beschichtung 11 versehenen Isolierkörper 10 gebildet, sondern durch einen metallischen Elektrodenkörper 40, der zugleich die Neutralelektrode darstellt. Die Zuleitung 20 der Aktivelektrode ist gemeinsam mit ihrer Isolierhülle 12 durch den Elektrodenkörper 40 bis über dessen distale Stirnfläche 41 hinaus geführt und trägt an ihrem distalen Ende die als Schneidschlinge 42 ausgeführte Aktivelektrode, wobei beide Fußbereiche 15, 16 der Schneidschlinge 42 in die Isolierhülle 12 münden und zumindest einer mit der Zuleitung 20 elektrisch verbunden ist. Die Schneidschlinge 42 kann durch den distalen Endabschnitt eines Drahts gebildet werden, der die Zuleitung 20 darstellt. Die Zuleitung 21 der Neutralelektrode ist innerhalb ihrer Isolierhülle 13 bis in den Elektrodenkörper 40 hinein geführt und dort mit diesem elektrisch kontaktiert. Wie in Fig. 6 angedeutet ist, kann die Zuleitung 21 sich durch den Elektrodenkörper 40 hindurch bis an dessen distale Stirnfläche 41 erstrecken. Im Übrigen ist die Elektrodenanordnung gemäß diesem Ausführungsbeispiel wie zuvor zu den Figuren 1 bis 5 beschrieben ausgebildet.

In Fig. 7 ist in einer Gesamtansicht beispielhaft ein Resektoskop mit einer in dieses eingesetzten erfindungsgemäßen Elektrodenanordnung dargestellt. Das Resektoskop 50 umfasst ein Arbeitselement 51 mit einem lang erstreckten Schaft 52, der zur Einführung in eine natürliche oder künstliche Körperöffnung bemessen ist, und einer Handhabe 53, die bei der Anwendung außerhalb des Körpers des Patienten verbleibt und die Anschlüsse, Bedienelemente und einen Verschiebemechanismus umfasst. Wie in Fig. 7 dargestellt, ist in den Schaft 52 des Arbeitselements eine Elektrodenanordnung 1 eingesetzt. Ferner ist aus proximaler Richtung in das Arbeitselement eine Endoskopoptik 54 eingesetzt und über eine Optikkupplung 55 mit dem Arbeitselement 51 verriegelt.

Die Elektrodenanordnung 1 ist derart in den Schaft 52 und weiter in die Handhabe 53 des Arbeitselements 51 eingeführt, so dass diese proximalseitig mit den Steckverbindern 6, 7 (s. Fig. 1) in einen Anschlussblock 56 eingesetzt und in diesem elektrisch kontaktiert und mechanisch arretiert ist. Der Anschlussblock 56 ist als verschiebbarer Schlitten ausgebildet, der mittels des Griffrings 57 in axialer Richtung relativ zum Schaft 52 verschoben werden kann. Am Anschlussblock 56 kann ein HF-Kabel zum Verbinden mit einer externen HF-Stromquelle angeschlossen werden. Über den Hebelmechanismus 58 ist der Anschlussblock 56 in proximaler Richtung federbelastet. Der Schaft 52 weist einen Außenschaft 59 sowie einen in Fig. 7 nur äußerst distal erkennbaren Innenschaft auf, die Kanäle zur Zuführung und Abführung von Spülflüssigkeit bilden. Hierfür sind Spülöffnungen 60 sowie Spülanschlüsse 61, 62 vorgesehen. Der Außenschaft 59 mit den Spülanschlüssen 61, 62 kann über eine Kupplung 63 mit den übrigen Elementen des Arbeitselements verbunden sein.

Bei der Anwendung wird der Schaft 52 mit der in diesen eingesetzten Elektrodenanordnung 1 bis zu einem Operationsgebiet in den Körper eines Patienten eingeführt. Der Anschlussblock 56 mit der Elektrodenanordnung 1 wird mittels des Griffrings 57 gegen die Federkraft in distaler Richtung bewegt, so dass der Elektrodenkörper 4 mit der Schneidschlinge 5 distalseitig aus dem Schaft 52 des Resektoskops 50 herausragen; diese Position ist in Fig. 7 gezeigt. Zum Schneiden von Gewebe wird sodann die Schneidschlinge 5 unter Beaufschlagung mit HF-Spannung mittels der Federkraft durch das zu durchtrennende Gewebe gezogen; in ihrer proximalen Endstellung ist die Elektrodenanordnung 1 ganz oder weitgehend in dem Außenschaft 59 aufgenommen (in Fig. 7 nicht gezeigt). Abgetrennte Gewebeteile können durch Spülung mit einer Spülflüssigkeit abgeführt werden. Anstelle der in Fig. 7 dargestellten Ausführungsform der Elektrodenanordnung 1 kann auch jede andere der erfindungsgemäßen und beispielhaft zuvor beschriebenen Elektrodenanordnungen verwendet werden.

Der Übersichtlichkeit halber sind nicht in allen Figuren alle Bezugszeichen dargestellt. Zu einer Figur nicht erläuterte Bezugszeichen haben eine entsprechende Bedeutung wie in den übrigen Figuren.

### Bezugszeichenliste

- 1: Elektrodenanordnung
- 2: Elektrodenträger
- 3: Elektrodenschaft
- 4: Elektrodenkörper
- 5: Schneidschlinge
- 6: Steckverbinder
- 7: Steckverbinder
- 8: Führungsklammer
- 9: Führungsklammer
- 10: Isolierkörper
- 11: Beschichtung
- 12: Isolierhülle
- 13: Isolierhülle
- 14: Stirnfläche
- 15: Fußabschnitt
- 16: Fußabschnitt
- 17: Schneidabschnitt
- 18: Querbohrung
- 19: Zwischenraum
- 20: Zuleitung
- 21: Zuleitung
- 30: Stab
- 31: Fußabschnitt
- 32: Kugel
- 40: Elektrodenkörper
- 41: Stirnfläche
- 42: Schneidschlinge
- 50: Resektoskop
- 51: Arbeitselement
- 52: Schaft
- 53: Handhabe
- 54: Endoskopoptik
- 55: Optikkupplung
- 56: Anschlussblock
- 57: Griffring
- 58: Hebelmechanismus
- 59: Außenschaft
- 60: Spülöffnungen, Rückfluss
- 61: Spülanschluss, Zuspülung
- 62: Spülanschluss, Abfluss
- 63: Kupplung

## Patentansprüche

1. Elektrodenanordnung für ein bipolares Resektoskop (50), umfassend einen lang erstreckten Elektrodenträger (2), eine Aktivelektrode, die an einem distalen Ende des Elektrodenträgers (2) angeordnet ist, und eine Neutralelektrode, wobei ein distaler Endabschnitt des Elektrodenträgers (2) als Elektrodenkörper (4, 40) ausgebildet ist, durch den eine Zuleitung (20) der Aktivelektrode geführt ist und wobei die Neutralelektrode durch den Elektrodenkörper (4, 40) oder einen Teilbereich des Elektrodenkörpers (4, 40) gebildet wird, wobei der Elektrodenträger (2) einen lang erstreckten Elektrodenschaft (3) umfasst, wobei der Elektrodenkörper (4, 40) in einer distalen Fortsetzung des Elektrodenschafts (3) angeordnet ist, wobei der Elektrodenschaft (3) und der Elektrodenkörper (4, 40) durch die Zuleitung (20) der Aktivelektrode und eine Zuleitung (21) der Neutralelektrode verbunden sind, **dadurch gekennzeichnet, dass** ein Querschnitt des Elektrodenkörpers (4, 40) zumindest näherungsweise einem Querschnitt des Elektrodenschafts (3) entspricht.

2. Elektrodenanordnung nach Anspruch 1, **dadurch gekennzeichnet, dass** der Elektrodenkörper (4) als Isolierkörper (10) mit einer elektrisch leitfähigen Schicht ausgebildet ist, die zumindest auf einen Teilbereich einer Oberfläche des Isolierkörpers (10) aufgebracht ist.

3. Elektrodenanordnung nach Anspruch 2, **dadurch gekennzeichnet, dass** der Isolierkörper (10) zumindest weitgehend aus einem keramischen oder einem anderen HF- und temperaturbeständigen Material besteht.

4. Elektrodenanordnung nach Anspruch 2 oder 3, **dadurch gekennzeichnet, dass** die elektrisch leitfähige Schicht eine elektronisch leitende, insbesondere metallische, Beschichtung (11) ist.

5. Elektrodenanordnung nach einem der Ansprüche 2 bis 4, **dadurch gekennzeichnet, dass** der Teilbereich der Oberfläche eine seitliche Oberfläche oder ein Teilbereich einer seitlichen Oberfläche des Elektrodenkörpers (4) ist.

6. Elektrodenanordnung nach einem der Ansprüche 2 bis 5, **dadurch gekennzeichnet, dass** der Elektrodenkörper (4) zumindest näherungsweise als ein Zylinder oder ein abgeflachter Zylinder ausgebildet ist, wobei der Teilbereich der Oberfläche zumindest ein Teilbereich einer Mantelfläche des Zylinders ist.

7. Elektrodenanordnung nach einem der Ansprüche 2 bis 6, **dadurch gekennzeichnet, dass** eine Zuleitung (21) der Neutralelektrode proximalseitig in den Isolierkörper (10) eingesetzt ist und dass sich die elektrisch leitfähige Schicht in eine bis zu der Zuleitung (21) der Neutralelektrode reichende Querbohrung (18) des Isolierkörpers (10) hinein erstreckt.

8. Elektrodenanordnung nach einem der Ansprüche 2 bis 7, **dadurch gekennzeichnet, dass** die Aktivelektrode als Schneidschlinge (5) mit einem ersten und einem zweiten Fußabschnitt (15, 16) und einem dazwischenliegenden Schneidabschnitt (17) ausgebildet ist, wobei der erste und der zweite Fußabschnitt (15, 16) an einer distalen Stirnfläche (14) des Isolierkörpers (10) ansetzen und nur der erste Fußabschnitt mit der Zuleitung (20) der Aktivelektrode verbunden ist.

9. Elektrodenanordnung nach einem der Ansprüche 2 bis 7, **dadurch gekennzeichnet, dass** die Aktivelektrode als Stabelektrode mit einem Stab (30) und einem Fußabschnitt (31) ausgebildet ist, wobei der Fußabschnitt (31) in eine distale Stirnfläche (14) des Isolierkörpers (10) eingesetzt und mit der Zuleitung (20) der Aktivelektrode verbunden ist.

10. Elektrodenanordnung nach Anspruch 9, **dadurch gekennzeichnet, dass** an dem vom Fußabschnitt (31) abgewandten Ende des Stabs (30) ein kugel-, halbkugel- oder scheibenförmiges Element angeordnet ist.

11. Elektrodenanordnung für ein bipolares Resektoskop nach Anspruch 1, **dadurch gekennzeichnet, dass** der Elektrodenkörper (40) als metallischer Elektrodenkörper (40) ausgebildet ist, der die Neutralelektrode bildet und durch den die Zuleitung (20) der Aktivelektrode isoliert geführt ist.

12. Elektrodenanordnung nach Anspruch 11, **dadurch gekennzeichnet, dass** die Aktivelektrode als Schneidschlinge (42) mit einem ersten und einem zweiten Fußabschnitt (15, 16) und einem dazwischenliegenden Schneidabschnitt (17) ausgebildet ist, wobei der erste und der zweite Fußabschnitt (15, 16) an einem distalen Ende der Zuleitung (20) der Aktivelektrode ansetzen.

13. Resektoskop, umfassend einen lang erstreckten Schaft (52), eine in diesen längs verschiebbar eingesetzte Elektrodenanordnung (1) und einen Verschiebemechanismus zum Verschieben der Elektrodenanordnung in Längsrichtung des Schafts, **dadurch gekennzeichnet, dass** die Elektrodenanordnung gemäß einem der vorhergehenden Ansprüche ausgebildet ist.

## Claims

1. Electrode arrangement for a bipolar resectoscope (50), comprising an elongate electrode carrier (2), an active electrode disposed at a distal end of the electrode carrier (2) and a neutral electrode, wherein a distal end section of the electrode carrier (2) is embodied as an electrode body (4, 40) through which a supply line (20) of the active electrode is guided and wherein the neutral electrode is formed by the electrode body (4, 40) or a portion of the electrode body (4, 40), wherein the electrode carrier (2) comprises an elongate electrode shaft (3), wherein the electrode body (4, 40) is disposed in a distal continuation of the electrode shaft (3), wherein the electrode shaft (3) and the electrode body (4, 40) are connected by the supply line (20) of the active electrode and a supply line (21) of the neutral electrode, **characterized in that** a cross section of the electrode body (4, 40) at least approximately corresponds to a cross section of the electrode shaft (3).

2. Electrode arrangement according to Claim 1, **characterized in that** the electrode body (4) is embodied as an insulation body (10) with an electrically conductive layer which is applied to at least a portion of a surface of the insulation body (10).

3. Electrode arrangement according to Claim 2, **characterized in that** the insulation body (10) consists at least predominantly of a ceramic or any other RF-resistant and temperature-resistant material.

4. Electrode arrangement according to Claim 2 or 3, **characterized in that** the electrically conductive layer is an electronically conducting, in particular metallic coating (11).

5. Electrode arrangement according to any one of Claims 2 to 4, **characterized in that** the portion of the surface is a lateral surface or a portion of a lateral surface of the electrode body (4).

6. Electrode arrangement according to any one of Claims 2 to 5, **characterized in that** the electrode body (4) is embodied at least approximately as a cylinder or a flattened cylinder, wherein the portion of the surface is at least one portion of a lateral surface of the cylinder.

7. Electrode arrangement according to any one of Claims 2 to 6, **characterized in that** a supply line (21) of the neutral electrode is inserted into the insulation body (10) on the proximal side and **in that** the electrically conductive layer extends into a transverse bore (18) of the insulation body (10) that reaches up to the supply line (21) of the neutral electrode.

8. Electrode arrangement according to any one of Claims 2 to 7, **characterized in that** the active electrode is embodied as a cutting loop (5) with a first and a second base section (15, 16) and a cutting section (17) lying therebetween, wherein the first and the second base section (15, 16) are positioned at a distal end face (14) of the insulation body (10) and only the first base section is connected to the supply line (20) of the active electrode.

9. Electrode arrangement according to any one of Claims 2 to 7, **characterized in that** the active electrode is embodied as a rod electrode with a rod (30) and a base section (31), wherein the base section (31) is inserted into a distal end face (14) of the insulation body (10) and connected to the supply line (20) of the active electrode.

10. Electrode arrangement according to Claim 9, **characterized in that** a spherical, hemispherical or disc-shaped element is disposed at the end of the rod (30) distant from the base section (31).

11. Electrode arrangement for a bipolar resectoscope according to Claim 1, **characterized in that** the electrode body (40) is embodied as a metallic electrode body (40) that forms the neutral electrode and the supply line (20) of the active electrode is guided therethrough in insulated fashion.

12. Electrode arrangement according to Claim 11, **characterized in that** the active electrode is embodied as a cutting loop (42) with a first and a second base section (15, 16) and a cutting section (17) lying therebetween, wherein the first and the second base section (15, 16) are positioned at a distal end of the supply line (20) of the active electrode.

13. Resectoscope comprising an elongate shaft (52), an electrode arrangement (1) inserted in longitudinally displaceable fashion in the latter and a displacement mechanism for displacing the electrode arrangement in the longitudinal direction of the shaft, **characterized in that** the electrode arrangement is embodied according to any one of the preceding claims.

## Revendications

1. Arrangement d'électrodes pour un résectoscope bipolaire (50), comprenant un porte-électrode (2) qui s'étend en longueur, une électrode active qui est disposée à une extrémité distale du porte-électrode (2) et une électrode neutre, une portion d'extrémité distale du porte-électrode (2) étant réalisée sous la forme d'un corps d'électrode (4, 40) à travers lequel passe une ligne d'alimentation (20) de l'électrode active et l'électrode neutre étant formée par le corps d'électrode (4, 40) ou une zone partielle du corps d'électrode (4, 40), le porte-électrode (2) comportant une tige d'électrode (3) qui s'étend en longueur, le corps d'électrode (4, 40) étant disposé dans une continuation distale de la tige d'électrode (3), la tige d'électrode (3) et le corps d'électrode (4, 40) étant reliés par la ligne d'alimentation (20) de l'électrode active et une ligne d'alimentation (21) de l'électrode neutre, **caractérisé en ce qu'**une section transversale du corps d'électrode (4, 40) correspond au moins approximativement à une section transversale de la tige d'électrode (3).

2. Arrangement d'électrodes selon la revendication 1, **caractérisé en ce que** le corps d'électrode (4) est réalisé sous la forme d'un corps isolant (10) doté d'une couche électriquement conductrice, laquelle est appliquée au moins sur une zone partielle d'une surface du corps isolant (10).

3. Arrangement d'électrodes selon la revendication 2, **caractérisé en ce que** le corps isolant (10) se compose au moins partiellement d'une céramique ou d'un autre matériau résistant aux HF et à la température.

4. Arrangement d'électrodes selon la revendication 2 ou 3, **caractérisé en ce que** la couche électriquement conductrice est un revêtement (11) électroniquement conducteur, notamment métallique.

5. Arrangement d'électrodes selon l'une des revendications 2 à 4, **caractérisé en ce que** la zone partielle de la surface est une surface latérale ou une zone partielle d'une surface latérale du corps d'électrode (4).

6. Arrangement d'électrodes selon l'une des revendications 2 à 5, **caractérisé en ce que** le corps d'électrode (4) est au moins approximativement réalisé sous la forme d'un cylindre ou d'un cylindre aplati, la zone partielle de la surface étant au moins une zone partielle d'une enveloppe du cylindre.

7. Arrangement d'électrodes selon l'une des revendications 2 à 6, **caractérisé en ce qu'**une ligne d'alimentation (21) de l'électrode neutre est introduite du côté proximal dans le corps isolant (10) et **en ce que** la couche électriquement conductrice s'étend à l'intérieur d'un trou transversal (18) du corps isolant (10) qui s'étend jusqu'à ligne d'alimentation (21) de l'électrode neutre.

8. Arrangement d'électrodes selon l'une des revendications 2 à 7, **caractérisé en ce que** l'électrode active est réalisée sous la forme d'une boucle de coupe (5) comprenant une première et une deuxième portion de pied (15, 16) et une portion de coupe (17) disposée entre celles-ci, la première et la deuxième portion de pied (15, 16) reposant contre une face frontale distale (14) du corps isolant (10) et seule la première portion de pied étant relié à la ligne d'alimentation (20) de l'électrode active.

9. Arrangement d'électrodes selon l'une des revendications 2 à 7, **caractérisé en ce que** l'électrode active est réalisée sous la forme d'une électrode en tige comprenant une tige (30) et une portion de pied (31), la portion de pied (31) étant insérée dans une face frontale distale (14) du corps isolant (10) et étant reliée à la ligne d'alimentation (20) de l'électrode active.

10. Arrangement d'électrodes selon la revendication 9, **caractérisé en ce qu'**un élément en forme de bille, de demi-sphère ou de disque est disposé à l'extrémité de la tige (30) à l'opposé de la portion de pied (31).

11. Arrangement d'électrodes pour un résectoscope bipolaire selon la revendication 1, **caractérisé en ce que** le corps d'électrode (40) est réalisé sous la forme d'un corps d'électrode (40) métallique lequel forme l'électrode neutre et à travers lequel passe de manière isolée la ligne d'alimentation (20) de l'électrode active.

12. Arrangement d'électrodes selon la revendication 11, **caractérisé en ce que** l'électrode active est réalisée sous la forme d'une boucle de coupe (42) comprenant une première et une deuxième portion de pied (15, 16) et une portion de coupe (17) disposée entre celles-ci, la première et la deuxième portion de pied (15, 16) reposant contre une extrémité distale de la ligne d'alimentation (20) de l'électrode active.

13. Résectoscope, comprenant une tige (52) qui s'étend en longueur, un arrangement d'électrodes (1) inséré dans celle-ci de manière à pouvoir coulisser longitudinalement et un mécanisme de coulissement destiné à faire coulisser l'arrangement d'électrodes dans la direction longitudinale de la tige, **caractérisé en ce que** l'arrangement d'électrodes est configuré selon l'une des revendications précédentes.
